# NEW EUROPEAN PATENT SPECIFICATION

(11) **EP 0 349 578 B2**
(45) Date of publication and mention of the opposition decision: **28.10.1998**
(45) Mention of the grant of the patent: 30.11.1994
(21) Application number: 88903058.1
(22) Date of filing: 02.03.1988
(51) Int. Cl.: C12P 21/08, C07K 17/00, C12N 1/00

(54) **Organism carrying a Single Chain Antibody Domain at its surface.**
Organismus als Träger für "Single Chain Antibody Domain (SCAD)".
Organism ayant un "Single Chain Antibody Domain (SCAD)" á l'exterieur.

(30) Priority: 02.03.1987 US 21046
(43) Date of publication of application: 10.01.1990
(73) Proprietor: Enzon Labs Inc., Piscataway New Jersey 08854 (US)
(72) Inventor: LADNER, Robert, Charles, Ijamsville, MD 27754 (US); GLICK, J., Leslie, Potomac, MD 20854 (US); BIRD, Robert, E., Kensington, MD 20895 (US)
(74) Representative: Sheard, Andrew Gregory
(86) International application number: US8800716
(87) International publication number: WO8806630

(56) References cited:
- EP-A- 120 694
- WO-A-91/17271
- WO-A-92/01047
- US-A- 4 593 002
- US-A- 4 603 112
- US-A- 4 704 692
- BIOTECHNOLOGY, vol. 4, no. 12, December 1986, pages 1041-1043, New York, US; A.KLAUSNER: "'Single-chain' antibodies become a reality"
- SCIENCE, vol. 242, 21th October 1988, pages 423-426; R.E. BIRD et al.: "Single-Chain antigen-binding proteins"
- McCafferty, J.,(1990), Nature, vol. 348:552-554.
- Clackson, T., et al. (1991), Nature, vol. 353:624-628.
- Barbas, C. F. et al., (1991), Proc. Natl. Acad. Sci. USA, vol. 88:7978-7982.
- Science, vol. 81, issued August, 1987, (Washington, D.C., USA), R. HUBER; pp. 702-703
- Biotechnology, vol. 3, issued April 1985, (New York, New York, USA) VALENZUELA et al.; pp. 323-326
- Biotechnology, vol. 4, issued April 1986, (New York, New York). J. Van Brunt, s. pp. 277-283
- J Mol Biol 156, 1982, p.93-112
- EMBO Journal 5(11), 1986, p.3029-3037
- FEMS Microbiology Reviews 32, 1985, p 3-38
- Biotechnology 3, 1985, p323

## Description

The present invention relates to the production of genetically engineered microoganisms and methods of preparation of binding molecules.

In vertebrates, antibody diversity arises from substitution of hypervariable loops into constant antibody frameworks. Each B-cell exhibits its own type of specificity on its surface. When an antigen binds to the surface antibody, the B-cell is stimulated to proliferate.

Monoclonal antibody production exploits this as follows: An animal is injected with a purified antigen. After several weeks, the spleen is removed from the animal and spleen cells are fused to myeloma cells. This produces hybridoma cells. These cells are plated and screened for binding to antigen. These cells can be grown in tissue culture and will produce quantities of a single antibody--a monoclonal antibody.

The gene for the antibody can be recovered and put into microorganisms. Genetic and protein engineering can be altered to obtain better binding, altered specificity, different antigenic behavior than that of the original protein or gene product.

Single-chain antibodies (SCA) (referred to in copending U.S. Patent Application Serial Nos. 902,971 and 902,970) are protein molecules which retain'the binding domain of antibodies but not the effector domains.

A. Klausner, *Biotechnology,* **4**: 1041-1043 (1986); refers to the production of single chain antibodies in *E. coli.*

US-A-4603112, US-A-4593002 and P. Valenzuela *et al, Biotechnology,* **3**: 323-326 (April 1985) all refer to the expression of immunogens on the surface of viruses for use as vaccines.

### Summary of the Invention

Thus according to the invention, there is provided a microorganism containing a recombinant gene wherein the product of the recombinant gene is a fusion of a polypeptide normally appearing on the outer surface of the organism with a single chain antibody domain (SCAD), said SCAD being present on the outer surface of said microorganism; wherein said recombinant gene is non-essential.

The invention also extends to a fusion polypeptide comprising a product normally appearing on the surface of a microorganism fused to a single chain antibody domain (SCAD).

The invention additionally extends to a method of preparing a microorganism containing a single chain binding molecule on the outer surface of the microorganism which comprises:
(1) isolating from a microorganism a first gene encoding for a cell polypeptide normally appearing on the surface of the microorganism, wherein said first gene is non-essential;
(2) inserting a second gene which encodes a single chain antibody domain into the first gene to form a recombinant fusion gene; and
(3) transforming a microorganism with the recombinant fusion gene.

Thus in the present invention, a genetically engineered microorganism is produced which displays on the outer surface of the microorganism the expression product of a gene which has been inserted. In one embodiment a SCA domain (SCAD) is displayed on the outside of a microorganism while the message for that particular SCA is inside that microorganism.

### Description of the Drawings

Figure 1, flow chart for production of microoganisms containing binding molecules on surface.

Figure 2, displaying SCAD on surface.

Figure 3, making diverse population of displayed SCADs.

Figure 4, selecting new SCA specificity.

Figure 5, detecting known antigens.

Figure 6, lambda assembly.

Figure 7, inserting SCAD into V genes.

### DESCRIPTION OF PREFERRED EMBODIMENTS

Any protein or antibody domain for which a gene can be isolated or constructed may be displayed on the outer surface of a microorganism into which the gene has been inserted. This is done by fusing the SCAD gene to the gene coding for a product which normally expresses on the surface of the microorganism; e.g., an envelope protein. The microorganism so produced may be easily isolated from microorganisms which do not contain the desired gene and express the gene product. The microorganisms may also serve as a solid substrate for the gene product. Prior to the present invention, once a microorganism which contained a desired gene had been produced, the microorganisms had to be grown and assayed for the production of the gene product. Then, the gene product had to be isolated, purified, and only then was it possible to couple it to a solid substrate. In one embodiment, the microorganism itself containing the gene product on its outer surface is the solid substrate with the desired gene product already attached and may be used as such.

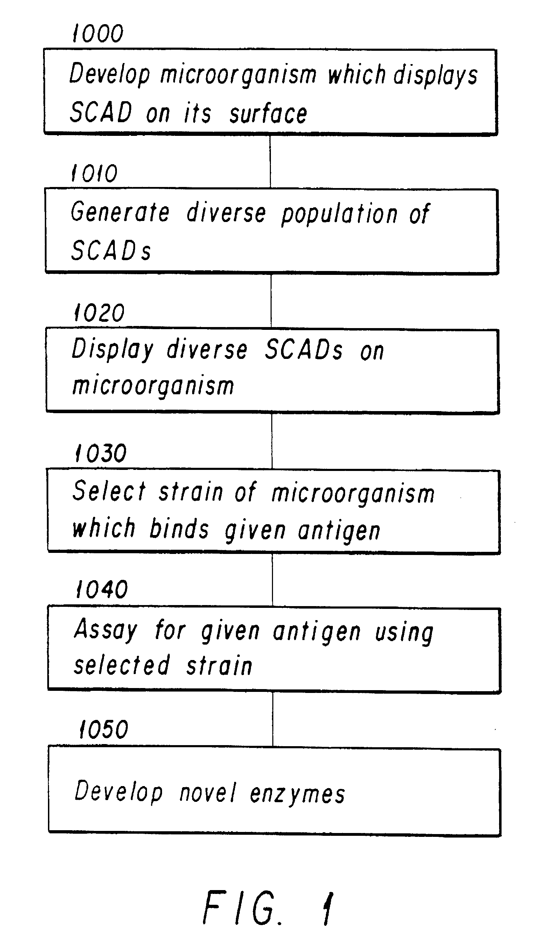

The present invention is depicted in general terms in the flow chart on Figure 1. One embodiment shown at step 1000 consists of producing microorganism. In this embodiment, a microorganism displays a gene product such as a SCAD on the surface of the microorganism. The next step (step 1010) consists of generating, from the one SCAD displayed and encoded in the microorganism, a diverse population of SCADs by varying the DNA sequence encoding the SCAD by mutation techniques. The new diverse SCADs generated in step 1010 are displayed on the surface of the microorganism (step 1020) and microorganism are selected based on the surface expressed SCAD which bind to given antigens (step 1030). The microorganisms selected in step 1030 may be used in assays for the given antigen or may be further selected according to the binding or enzymatic characteristics of the gene product expressed on the surface.

Once any SCAD has been displayed on the surface of a microorganism, a large population of different SCADs can be generated by in vivo DNA synthesis, step 1010, and each cell or virion can display its own SCAD specificity, step 1020. Antigen binding to the displayed SCAD can be used to select those microorganisms harboring genes for SCADs which will bind antigen, step 1030. Once a strain of microorganisms is selected for antigen binding, it can be used as a sensitive assay for that antigen, step 1040. In step 1050, the ability to refine antigen binding is used to generate novel enzymes.

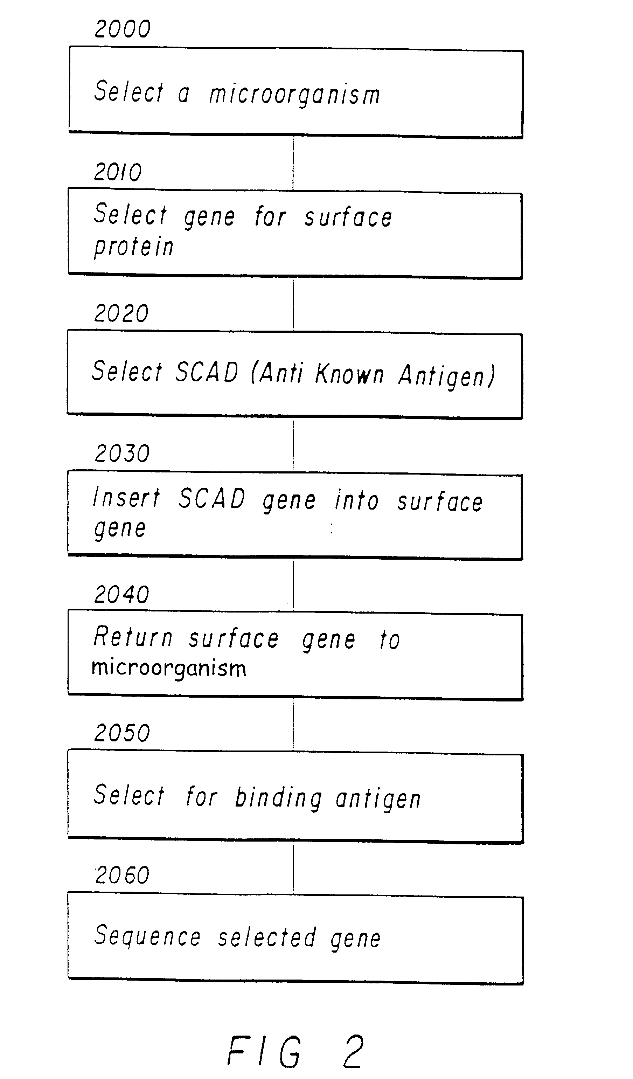

The steps needed to achieve the construction of a micro-organism which displays SCAD are shown in the flow chart of Figure 2. In step 2000, a microorganism is selected. In step 2010, a gene within that microorganism is selected; the gene must be one coding for a protein which is displayed on the cell or virion surface. Preferably, the gene should not be essential to the microorganism. In step 2020, the gene for a SCAD to some known antigen is introduced into the selected gene, and in step 2030, this population of modified genes is put back into the microorganism. In step 2040 the genes are expressed, and in step 2050 the microorganisms are selected for binding to immobilized antigen. In step 2060, the gene is sequenced to determine which insertion was fruitful.

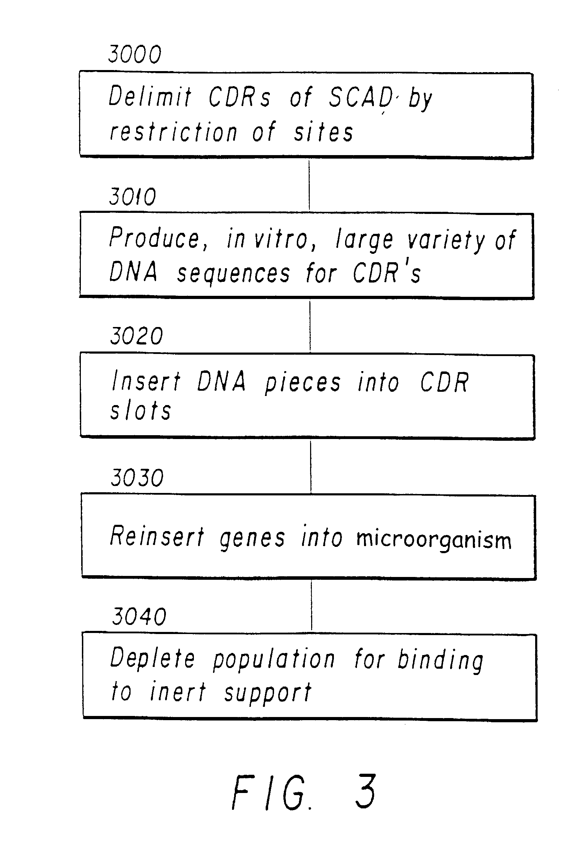

The steps needed to create a diverse population of SCADs displayed on the surface of a microorganism are illustrated in the flow chart on Figure 3. In step 3000, the Combining Determining Regions (CDRs) of the SCA are bounded by restriction sites. In step 3010, a large variety of DNA sequences are produced. Each sequence should begin with one of the restriction sequences and end with the corresponding restriction site. Between these sites should come any constant residues which are included to facilitate placement of restriction sites plus an integral number of triplets. The number of triplets can be varied within the bounds set by:
1. Analysis of sequences of natural antibodies with similar framework;
2. Computer modeling of the framework;
3. Trial and error.
In step 3020, these DNA sequences are inserted into the appropriate slots in the SCAD gene. In step 3030, these genes are reinserted into the microorganism and grown. The microorganism now contains a diversity of SCA specifications, each cell displaying its own particular SCAD. In step 3040, this population is passed over the inert suppport which will be used to support antigen. This step removes those microorganisms which bind to the inert support even without antigen.

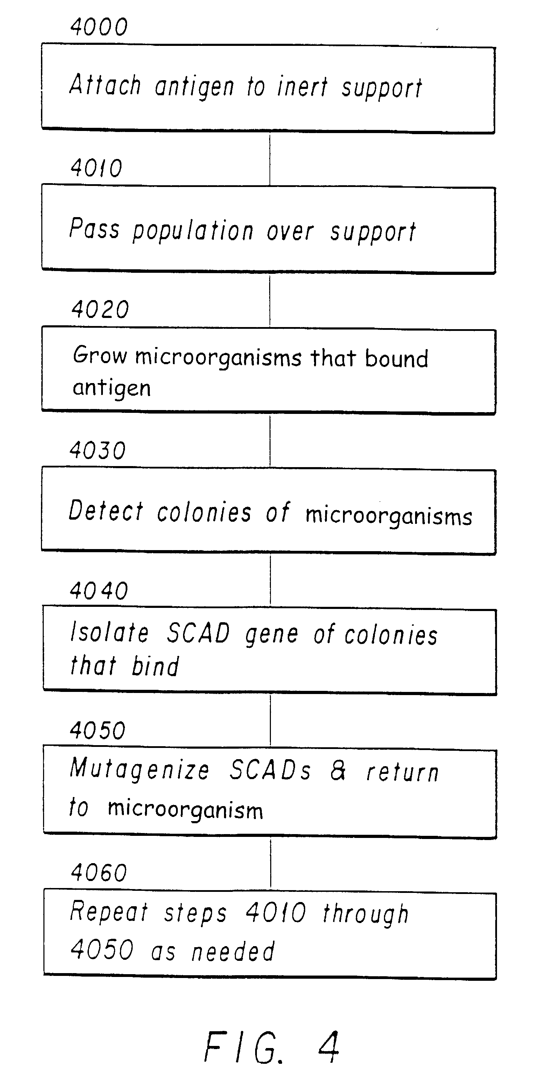

In step 4000 in Figure 4, the antigen is attached to an inert support. In step 4010, the population of microorganisms prepared in steps 3000 to 3040 is passed over the supported antigen. Microorganisms not binding pass through. In step 4020, the microorganisms bound to the support are allowed to grow. In step 4030, colonies are found and sampled. In step 4040, the genes of several isolates are sequenced. In step 4050, the SCAD gene of selected microorganisms are mutagenized. In step 4060, step 4010 through 4050 are repeated with the mutagenized colonies. In step 4060, by washing more stringently, a SCA colony with maximal binding is obtained. Step 4060 can be repeated until suitable binding is obtained.

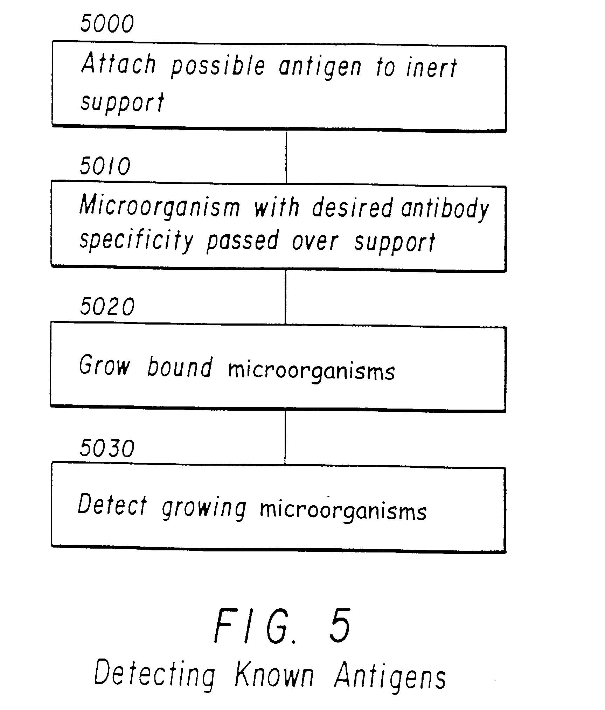

The present invention is also useful for the detection and quantification of known antigens. In step 5000 of Figure 5, a sample with unknown amount of an antigen is attached to an inert support. In step 5010, the strain of microorganism derived in step 4060 and displaying a SCAD against the antigen is passed over the inert support. In step 5020, the bound microorganisms are allowed to grow. In step 5030, points of growth are detected, the amount of growth quantitates the amount of antigen.

Enzymes, particularly degradative enzymes, work by stabilizing the transition state of a reaction. Chemical theory suggests the shape of the transition states of many reactions. For example, the carbonyl carbons of esters of carboxylic acids are trigonal planar. The transition state for hydrolysis or transesterification is almost certainly tetrahedral. It has recently been demonstrated that a monoclonal antibody against a phosphate ester (which is tetrahedral) is also an esterase.

Monoclonal antibody technology has many shortcomings for this task:
1. Slow turnaround
2. Difficulty in refining antibody
3. Inappropriate for highly toxic chemicals
4. Inappropriate for metabolites
The method described above can be applied to produce such enzyme-like binding molecules. It will be limited only in the ability to invent antigens which resemble the transition states of reactions wished to catalyze.

For example, to catalyse the transesterification 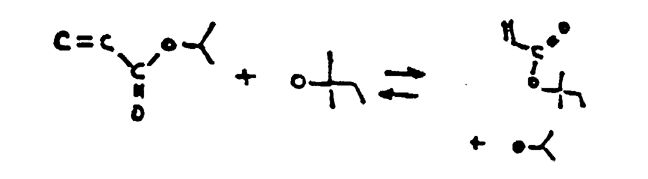 one can raise a SCA against

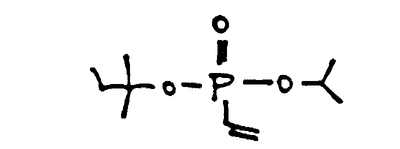

Having now generally described the invention, the same may further be understood by reference to the following expressly stated.

### Example 1

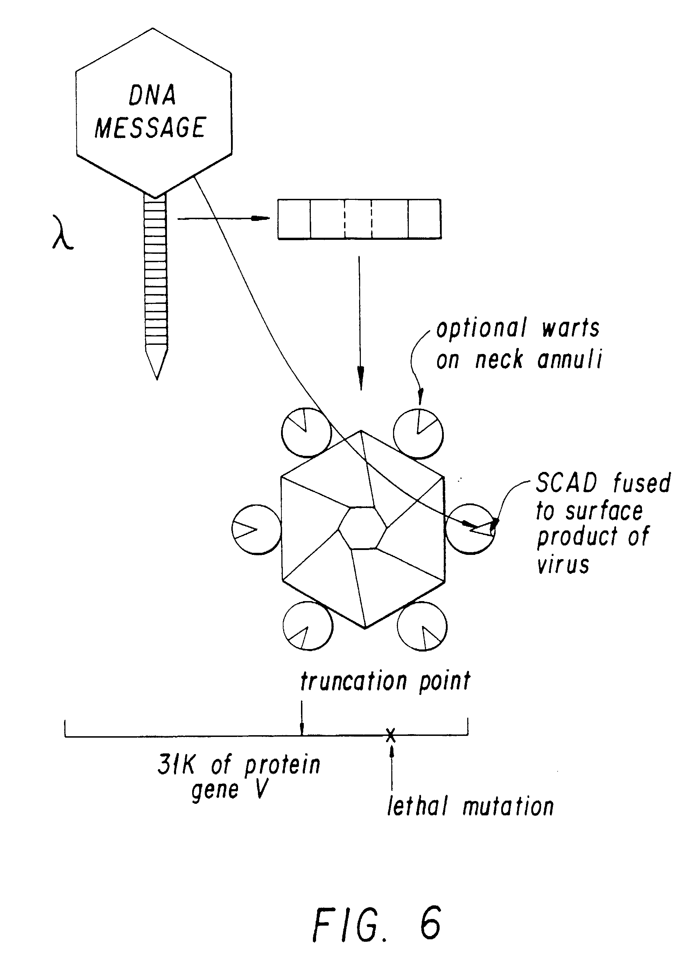

The preferred embodiment utilizes the bacteriophage lambda. The gene V of lambda generates a protein which assembles to form the neck of lambda. First gene product V (gpV) forms hexameric annuli, then 32 of these annuli stack on the nose cone to form the neck (Figure 6). Finally, the neck joins the head which contains the DNA.

gpV is a protein of molecular weight 31K. Wild-type lambda have small protuberances on the outside of the neck annuli. Mutants have been isolated in which as much as 13K of gpV is absent. These mutants are viable, though temperature sensitive. The mutants are those wherein shortened gpV lack the protuberances on the neck annuli. Genetics indicates that the deletion is from the carboxy end of gene V.

SCAs made so far contain the four cysteine residues found in all V^{H} and V¹ domains of natural antibodies. Natural antibodies are secreted and fold in the oxidizing environment of serum. The interior of cells is a reducing environment; thus, one would not expect disulfide bonds to form. The sulfhydryl groups of cysteine lie only 2.0 A apart when a disulfide forms. If the disulfide is reduced, the sulfur atoms should lie 4.0 A apart. Thus, reduced cysteines will greatly destabilize folding of a SCA. Therefore, to get proper folding of SCAD inside a cell, one mutates the SCAD gene to change all or some of the CYS's to SER, THR, ALA, or GLY. In one embodiment, the SCA is against bovine growth hormone (BGH).

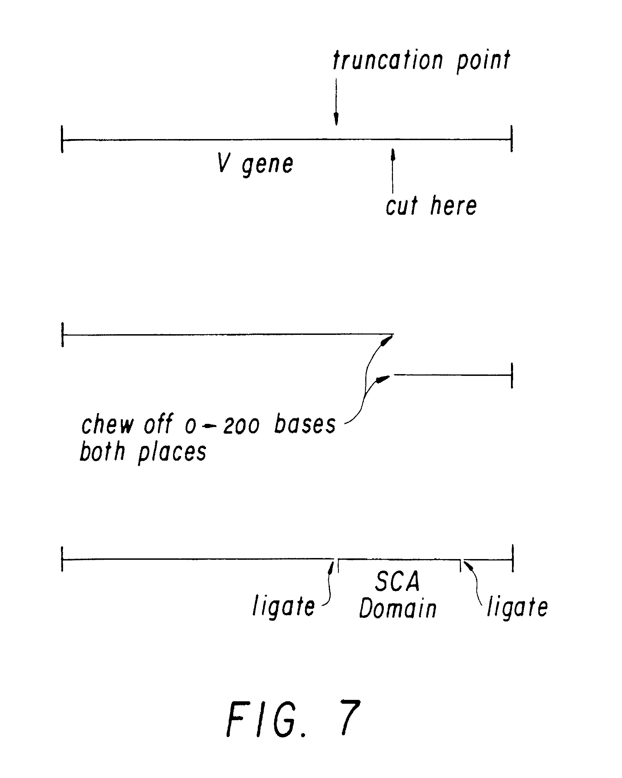

The V gene of lambda is shown in Figure 7. Genetics indicates that the domain responsible for the warts on the neck lies in the 300 to 400 last base pairs to the right. One cuts the gene at some point in this region, preferably 200 bases from the right end. A random number of bases on either side, up to 200 bases is removed. The SCAD (antiBGH) is inserted and put back into a lysogenic strain of E. coli. In the preferred embodiment, the lambda contains a highly beneficial gene for the E. coli.

The E. coli is induced. The lambda progeny is passed over a support holding BGH. The E. coli is contacted with the support. The coli should be deficient in a way that the beneficial gene in the lambda will complement. For example the coli could be drug-sensitive and lambda will carry drug resistance. The corresponding antibiotic in the medium puts the coli under selective pressure so that only those cells infected by lambda will grow. Only those lambda which bound antigen and stuck to the support are available.

## Claims

1. A micro-organism containing a recombinant gene wherein the product of the recombinant gene is a fusion of a polypeptide normally appearing on the outer surface of the organism with a single chain antibody domain (SCAD), said SCAD being present on the outer surface of said micro-organism; wherein said recombinant gene is non-essential.

2. A micro-organism as claimed in claim 1 wherein the SCAD possesses enzymatic activity.

3. A micro-organism according to any preceding claim wherein the polypeptide normally appearing on the surface of the micro-organism is an envelope protein.

4. A fusion polypeptide comprising a product normally appearing on the surface of a micro-organism fused to a single chain antibody domain (SCAD).

5. A fusion polypeptide as claimed in claim 4 wherein the SCAD possesses enzymatic activity.

6. A fusion polypeptide according to claim 4 or 5 wherein the product is an envelope protein.

7. A method of preparing a micro-organism containing a single chain binding molecule on the outer surface of the micro-organism which comprises:
(1) isolating from a micro-organism a first gene encoding for a cell polypeptide normally appearing on the surface of the micro-organism, wherein said first gene is non-essential;
(2) inserting a second gene which encodes a single chain antibody domain into the first gene to form a recombinant fusion gene; and
(3) transforming a micro-organism with the recombinant fusion gene.

8. A method as claimed in claim 7 wherein the single chain binding molecule possesses enzymatic activity.

## Patentansprüche

1. Mikroorganismus, welcher ein rekombinantes Gen enthält, wobei das Produkt des rekombinanten Gens eine Fusion eines normalerweise an der Außenfläche des Organismus aufscheinenden Polypeptids mit einer einzelkettigen Antikörperdomäne ("Single Chain Antibody Domain") (SCAD) ist, wobei die genannte SCAD auf der Außenfläche des Organismus vorhanden ist; und wobei das genannte rekombinante Gen ein nicht-essentielles ist.

2. Organismus nach Anspruch 1, worin die SCAD enzymatische Aktivität besitzt.

3. Organismus nach einem der vorangehenden Ansprüche, worin das Polypeptid, das normalerweise an der Oberfläche des Organismus aufscheint, ein Hüllenprotein ist.

4. Fusionspolypeptid, welches ein Produkt enthält, das normalerweise an der Oberfläche eines Organismus aufscheint und welches mit einer einzelkettigen Antikörperdomäne ("Single Chain Antibody Domain") (SCAD) verschmolzen ist.

5. Polypeptid nach Anspruch 4, worin die SCAD enzymatische Aktivität besitzt.

6. Fusionspolypeptid nach Anspruch 4 oder 5, worin das Produkt ein Hüllenprotein ist.

7. Verfahren zur Herstellung eines Organismus, der ein Einzelketten-Bindungsmolekül an der Außenfläche des Organismus enthält, welches umfaßt:
(1) das Isolieren eines ersten Gens von einem Organismus, das für ein Zellpolypeptid kodiert, welches normalerweise an der Oberfläche des Organismus aufscheint, wobei das genannte erste Gen ein nicht-essentielles ist.
(2) das Einsetzen eines zweiten Gens, das für eine einzelkettige Antikörperdomäne ("Single Chain Antibody Domain") kodiert, in das erste Gen zur Bildung eines rekombinanten Fusionsgens;
(3) die Transformation eines Organismus mit dem rekombinanten Fusionsgen.

8. Verfahren nach Anspruch 7, worin das erste Gen ein nicht-essentielles ist, und/oder das Einzelketten-Bindungsmolekül. enzymatische Aktivität besitzt.

## Revendications

1. Micro-organisme contenant un gène recombiné, dans lequel le produit du gène recombiné est une fusion d'un polypeptide apparaissant normalement sur la surface extérieure de l'organisme avec un domaine d'anticorps à chaîne unique (SCAD), le SCAD étant présent sur la surface extérieure du micro-organisme; dans lequel le gène recombiné est non essentiel.

2. Micro-organisme suivant la revendication 1, dans lequel le SCAD possède une activité enzymatique.

3. Micro-organisme suivant l'une quelconque des revendications précédentes, dans lequel le polypeptide apparaissant normalement sur la surface du micro-organisme est une protéine d'enveloppe.

4. Polypeptide de fusion comprenant un produit apparaissant normalement sur la surface d'un micro-organisme fusionné à un domaine d'anticorps à chaîne unique (SCAD).

5. Polypeptide suivant la revendication 4, dans lequel le SCAD possède une activité enzymatique.

6. Polypeptide de fusion suivant la revendication 4 ou 5, dans lequel le produit est une protéine d'enveloppe.

7. Procédé de préparation d'un micro-organisme contenant une molécule fixant une chaîne unique sur la surface extérieure du micro-organisme, qui comprend :
(1) l'isolement d'un micro-organisme, d'un premier gène codant pour un polypeptide cellulaire apparaissant normalement sur la surface du micro-organisme, dans lequel le premier gène est non essentiel;
(2) l'insertion d'un deuxième gène qui code un domaine d'anticorps à chaîne unique dans le premier gène pour former un gène de fusion recombiné, et
(3) la transformation d'un micro-organisme avec le gène de fusion recombiné.

8. Procédé suivant la revendication 7, dans lequel la molécule fixant une chaîne unique possède une activité enzymatique.
